(19) Europäisches Patentamt

European Patent Office

Office européen des brevets

(11) **EP 1 228 022 B1**

(12) **EUROPÄISCHE PATENTSCHRIFT**

(45) Veröffentlichungstag und Bekanntmachung des Hinweises auf die Patenterteilung:
**09.06.2004 Patentblatt 2004/24**

(21) Anmeldenummer: 00974475.6

(22) Anmeldetag: **28.10.2000**

(51) Int Cl.$^7$: **C07C 17/02**, C07C 19/045

(86) Internationale Anmeldenummer:
**PCT/EP2000/010630**

(87) Internationale Veröffentlichungsnummer:
**WO 2001/034542 (17.05.2001 Gazette 2001/20)**

(54) **VERFAHREN UND VORRICHTUNG ZUR NUTZUNG DER BEI DER HERSTELLUNG VON 1,2-DICHLORETHAN ANFALLENDEN REAKTIONSWÄRME**

METHOD AND DEVICE FOR EXPLOITING HEAT RESULTING FROM THE PRODUCTION OF 1,2-DICHLOROETHANE

PROCEDE ET DISPOSITIF PERMETTANT D'UTILISER LA CHALEUR DE REACTION DEGAGEE LORS DE LA PRODUCTION DE 1,2-DICHLORETHANE

(84) Benannte Vertragsstaaten:
**AT BE CH CY DE DK ES FI FR GB GR IE IT LI LU MC NL PT SE**

(30) Priorität: **09.11.1999 DE 19953762**

(43) Veröffentlichungstag der Anmeldung:
**07.08.2002 Patentblatt 2002/32**

(73) Patentinhaber: **Uhde GmbH**
**44141 Dortmund (DE)**

(72) Erfinder:
• **BENJE, Michael**
**64289 Darmstadt (DE)**
• **PORSCHA, Peter**
**65779 Kelkheim (DE)**
• **VON EGELSTEIN, Stefan**
**63762 Grossostheim (DE)**

(56) Entgegenhaltungen:
EP-A- 0 075 742          DE-A- 4 039 960
DE-A- 19 641 562        DE-C- 19 916 753
US-A- 4 347 391          US-A- 4 873 384

**Beschreibung**

Die Erfindung richtet sich auf ein Verfahren und eine Vorrichtung zur Herstellung von 1,2-Dichlorethan, im folgenden als EDC bezeichnet, welches überwiegend als Zwischenprodukt der Herstellung von monomerem Vinylchlorid, im folgenden als VCM bezeichnet, dient, woraus letztlich Polyvinylchlorid, PVC, hergestellt wird. Bei der Umsetzung von EDC zu VCM entsteht Chlorwasserstoff HCl, EDC wird daher bevorzugt aus Ethen $C_2H_4$ und Chlor $Cl_2$ derart hergestellt, dass hinsichtlich des bei den Umsetzungen erzeugten und verbrauchten Chlorwasserstoffes HCl eine ausgewogene Bilanz entsprechend den folgenden Reaktionsgleichungen erreicht wird:

$$Cl_2 + C_2H_4 \rightarrow C_2H_4Cl_2 \text{ (Rein-EDC)} + 180 \text{ kJ/Mol} \tag{1}$$

$$C_2H_4Cl_2 \text{ (Spalt-EDC)} \rightarrow C_2H_3Cl \text{ (VCM)} + HCl - 71 \text{ kJ/Mol} \tag{2}$$

$$C_2H_4 + 2 HCl + \tfrac{1}{2} O_2 \rightarrow C_2H_4Cl_2 \text{ (Roh-EDC)} + H_2O + 238 \text{ kJ/Mol} \tag{3}$$

Das Verfahren zur Herstellung von VCM mit ausgewogener HCl-Bilanz, im folgenden kurz "ausgewogenes VCM-Verfahren" genannt, besitzt:

- eine Direktchlorierung, in der aus Ethen $C_2H_4$ und Chlor $Cl_2$ der eine Teil des benötigten EDC erzeugt wird und als sogenanntes Rein-EDC abgegeben wird; die Verwertung der bei dieser Direktchlorierung erzeugten Reaktionswärme ist zentraler Bestandteil der Erfindung;
- eine Oxichlorierung, in der aus Ethen $C_2H_4$, Chlorwasserstoff HCl und Sauerstoff $O_2$ der andere Teil des EDC erzeugt wird und als sogenanntes Roh-EDC abgegeben wird;
- eine fraktionierende EDC-Reinigung, in der das Roh-EDC zusammen mit dem aus der VCM-Fraktionierung rezirkulierten Rück-EDC von den in der Oxichlorierung und von den in der EDC-Pyrolyse gebildeten Nebenprodukten befreit wird, um ein für den Einsatz in der EDC-Pyrolyse geeignetes, sogenanntes Feed-EDC zu gewinnen; die Nutzung der Reaktionsabwärme der Direktchlorierung in der EDC-Reinigung ist zentraler Bestandteil der Erfindung;
- eine EDC-Pyrolyse, in der das Rein-EDC mit dem Feed-EDC zusammengeführt und in der das dann Spalt-EDC genannte Gemisch thermisch gespalten wird; das erhaltene Spaltgas enthält VCM, Chlorwasserstoff HCl und nichtumgesetztes EDC sowie Nebenprodukte;
- eine VCM-Fraktionierung, in der das als Produkt gewünschte Rein-VCM aus dem Spaltgas abgetrennt und die anderen wesentlichen Spaltgasbestandteile Chlorwasserstoff HCl und nichtumgesetztes EDC als Wertstoffe gesondert zurückgewonnen und als wiederverwertbarer Einsatz als Rück-HCl bzw. Rück-EDC im ausgewogenen VCM-Verfahren rezirkuliert werden.

Die in der EDC-Pyrolyse im Spaltgas entstehenden Begleitstoffe setzen die Produkt-Reinheit des VCM herab. Die Reinigung des VCM durch Entfernung der Begleitstoffe ist dementsprechend aufwendig. Daher besteht bei den Anwendern des ausgewogenen VCM-Verfahrens durchaus das Bestreben, den mit der Reinigung des VCM in der Fraktionierung verbundenen Aufwand zu verringern. Eine Überlegung ist darauf gerichtet, die Entstehung der missliebigen Begleitstoffe in der EDC-Pyrolyse hinsichtlich Art und Menge zu begrenzen. Auf diesem Wege entsteht die Forderung, ein weitestgehend von Verunreinigungen befreites Spalt-EDC in der EDC-Pyrolyse einzusetzen. Die Verunreinigungen im Spalt-EDC entstehen zu einem Teil bei der Herstellung des Rein-EDC, zum anderen Teil werden sie mit dem zugemischten Rück-EDC eingetragen. Einige der verunreinigenden Stoffe sind Precursoren bei der EDC-Pyrolyse für die Entstehung der zusätzlichen Begleitstoffe im Spaltgas. Daher gilt es, mit dem Spalt-EDC so wenig wie möglich an Precursoren in die EDC-Pyrolyse einzuspeisen.

Es sind daher in der Zwischenzeit seit dem Aufkommen des ausgewogenen VCM-Verfahrens eine Anzahl von Vorschlägen bekannt geworden, wie die entsprechenden und nachteiligen Nebenprodukte und/oder Begleitstoffe vermieden bzw. gegebenenfalls abgereinigt werden sollen. Bekanntlich wird ein Teil des für die EDC-Pyrolyse benötigten EDC nach dem Direktchlorierungsverfahren durch Umsetzung von Ethen $C_2H_4$ und Chlor $Cl_2$ in flüssigem EDC hergestellt. Die Reaktion ist exotherm und es ist eine erhebliche Kühlleistung zur Aufrechterhaltung der Reaktionstemperatur erforderlich. Die Direktchlorierungsreaktion findet in einem umlaufenden Strom des Reaktionsproduktes EDC in Gegenwart einer Lewis-Säure (meist Eisen-(III)-chlorid) sowie eines Inhibitors (meist Sauerstoff) statt. Die bekannten Reaktionssysteme unterscheiden sich nach der Art der Erzeugung dieses Umlaufstromes, es werden nämlich Systeme mit Naturumlauf und Systeme mit Zwangsumlauf eingesetzt.

Ein solches System mit Naturumlauf ist beispielsweise in der Schrift DE 24 27 045 beschrieben. Die Reaktanden werden hier am unteren Ende des Steigrohrs eines Naturumlauf-Schlaufenreaktors zugegeben. Der Reaktorinhalt beginnt im oberen Teil des Steigrohrs zu sieden und die dampfförmigen Reaktionsprodukte werden direkt in den Sumpf einer Rektifikationskolonne zu deren Direktbeheizung eingespeist.

Das Reaktionsmedium EDC lässt sich auch im Zwangsumlauf führen. Ein System dieser Art ist z.B. in der Schrift DE 40 29 314 A1 beschrieben. Das Chlor wird durch einen als Injektor ausgebildeten Flüssigkeitsstrahl-Gasverdichter angesaugt und löst sich teilweise im Reaktionsmedium. Stromabwärts wird dann das Ethen durch einen konventionellen Gasverteiler zunächst in Form größerer Gasblasen zugemischt. Der gesamte Strom passiert danach einen statischen Mischer, in dem größere Gasblasen dispergiert werden, um die Auflösung und die damit gekoppelte Reaktion zu erleichtern. Bei einem System dieser Art siedet der Inhalt des eigentlichen Reaktors nicht; die EDC-Produktion wird durch Entspannungsverdampfung eines Teilstromes des EDC-Reaktionsmediums gasförmig abgezogen. Die Wärmerückgewinnung geschieht bei diesem System mittels fühlbarer Wärme des EDC-Reaktionsmediums, wie z.B. in der Schrift EP 0 075 742 B1 beschrieben. Der flüssige EDC-Umlaufstrom durchströmt hier einen oder mehrere als Sumpferhitzer dienende Naturumlaufverdampfer und gibt dabei fühlbare Wärme an den siedenden Inhalt des Kolonnensumpfes ab.

In der Schrift DE 40 29 314 A1 wird ein Verfahren zur Direktchlorierung beschrieben, bei dem die Bildung der gesamten chlorierten Nebenprodukte im EDC, welches im Schlaufenreaktor mittels eines $NaFeCl_4$-Katalysators erzeugt wird, unter einem Wert von 500 ppm liegt. Dieser Reinheitsgrad des EDC ist normalerweise hoch genug, dass es ohne weitere zwischengeschaltete Reinigung der EDC-Pyrolyse zugeführt werden kann. Ein derart hoher Reinheitsgrad ist von größter Wichtigkeit, um damit einerseits Nebenreaktionen, die zur Verschmutzung der Pyrolyserohre führen, zu unterdrücken und andererseits auch noch eine hohe Reinheit des VCM zu erreichen. Deshalb wird auf jeden Fall das nicht in der EDC-Pyrolyse umgesetzte EDC, das sogenannte Rück-EDC, sowie das EDC aus der Oxichlorierung, das sogenannte Roh-EDC, in einer energieaufwendigen, destillativen EDC-Fraktionierung gereinigt, bevor es in die EDC-Pyrolyse eingespeist wird. Sofern ein geringerer Gehalt an Verunreinigungen als 500 ppm im EDC gefordert wird, ist es erforderlich, auch das Rein-EDC aus der Direktchlorierung in einer zusätzlichen destillativen Reinigungsstufe weiter aufzureinigen.

Die Reinigung aller EDC-Ströme geschieht üblicherweise destillativ in der deshalb so genannten EDC-Destillation. Zwecks EDC-Reinigung wird das Roh-EDC aus der Oxichlorierung und das nicht umgesetzte EDC aus der EDC-Pyrolyse in einer energieaufwendigen EDC-Destillation gereinigt. Falls erforderlich kann auch das in der Direktchlorierung hergestellte Rein-EDC zusammen mit dem Roh-EDC und dem Rück-EDC in der EDC-Reinigung auf destillativen Wege gereinigt werden. Der zu reinigende EDC-Strom wird zunächst in eine Fraktionierkolonne zur Abtrennung von Wasser und Leichtsiedern, im folgenden als "Leichtsiederkolonne" bezeichnet, gegeben, danach wird das teilgereinigte EDC, welches noch Hochsieder enthält, als Sumpfablauf aus der Leichtsiederkolonne abgezogen und einer Fraktionierkolonne zur Abtrennung von Hochsiedern, im folgenden als "Hochsiederkolonne" bezeichnet, zugeführt. Anstelle der Leichtsiederkolonne kann auch eine Reihe von getrennten Kolonnen eingesetzt werden

Das von der EDC-Pyrolyse kommende, nicht umgesetzte Rück-EDC enthält ebenfalls Hochsieder und wird gleichfalls der Hochsiederkolonne zugeführt. In der Hochsiederkolonne werden die zugeführten Stoffströme zusammen destilliert. Eine erste Teilmenge von gereinigten und weitgehend von den Hochsiedern befreiten EDC-Brüden wird am Kopf der Hochsiederkolonne abgezogen, im Wärmeübertrager kondensiert und als reines EDC flüssig gewonnen. Im Sumpf der Hochsiederkolonne reichem sich die Hochsieder an. Eine verstärkte Reinigung des Sumpfablaufes der Hochsiederkolonne ist möglich, indem der Sumpfablauf einer als "Vakuumkolonne" bezeichneten zweiten Hochsiederkolonne zugeführt wird. Eine zweite Teilmenge von gereinigten und weitgehend von den Hochsiedern befreiten EDC-Brüden wird am Kopf der Vakuumkolonne abgezogen, im Wärmeübertrager kondensiert und als reines EDC flüssig gewonnen. Beide Teilmengen bilden nach ihrer Zusammenführung das Spalt-EDC. Der Sumpfabzug der Vakuumkolonne besteht im wesentlichen aus Hochsiedern und einem kleinem Anteil EDC und wird entsorgt.

In der Praxis zeigt sich, dass der Direktchlorierungsreaktor zu den größten Verbrauchern für Kühlmedien und die Hochsiederkolonne wie auch die Vakuumkolonne zu den größten Verbrauchern für Heizenergie innerhalb einer EDC/VCM-Anlage nach dem ausgewogenen VCM-Verfahren gehören. Aus wirtschaftlichen Erwägungen sind deshalb verschiedene Wege vorgeschlagen worden, um den Energieverbrauch zu verringern, wobei die Vorschläge sich mit der Beheizung der Kolonnen befassten. Bei diesen Vorschlägen wird entweder die Reaktionswärme der Direktchlorierung zur Beheizung der EDC-Destillation durch direkte oder indirekte Beheizung ausgenutzt oder aber lediglich vergleichsweise niederwertige Heizenergie eingespart, indem das Prinzip Rektifikation mit Brüdenverdichtung bei der destillativen EDC-Reinigung angewendet wird. Den vorgeschlagenen Verfahren haften jedoch Nachteile an, die im folgenden kurz erläutert werden.

Bei der direkten Beheizung werden die im Direktchlorierungsreaktor gebildeten Produktdämpfe unmittelbar in den Sumpf der Hochsiederkolonne eingeleitet, wie es z.B. in den Schriften DE 29 35 884 und DE 24 27 045 beschrieben wird. Der Durchmesser der Hochsiederkolonne und die Fläche ihres Rücklaufkondensators werden dann sehr groß, da bei der exothermen Siedereaktion ohne Fremdkühlung in Relation zum in der Direktchlorierung neu gebildeten EDC eine mehrfache Menge an EDC verdampft und zusätzlich zu den anderen EDC-Strömen in der Hochsiederkolonne rektifiziert werden muss. Da gleichzeitig vom Sumpf der Hochsiederkolonne flüssiges EDC- ebenfalls die mehrfache Menge des im Direktchlorierungsreaktor neu gebildeten EDC- zurück zum Reaktor gefahren werden muss, stellt sich gezwungenermaßen ein erhöhtes Niveau an höhersiedenden Nebenprodukten im Reaktor ein, welches sich einerseits negativ auf die Katalysatorwirksamkeit auswirkt und andererseits die Nebenproduktbildung fördert, was letztlich zu Ausbeuteeinbußen führt.

Auf Grund der oben geschilderten Zusammenhänge kann die Reaktionswärme der Direktchlorierung auch nicht für die direkte Beheizung der Vakuumkolonne in der EDC-Reinigung genutzt werden, da hier die Konzentration an Hochsiedern im Sumpf mit 90% noch weit größer als in der Hochsiederkolonne ist.

Bei der indirekten Beheizung kann man zwischen flüssigem und dampfförmigem EDC als Heizmedium wählen. Es ergeben sich die folgenden Konsequenzen, wie sie in den Schriften DE 196 41 562, DE 41 33 810 A1, DE 40 39 960 A1, DE 36 04 968 und EP 0 075 742 B1 beschrieben sind:

- Wenn bei dem vorhandenen Stoffsystem Naturumlaufverdampfer in den verbundenen Kolonnen eingesetzt werden, ist für eine einwandfreie Beheizung eine treibende Temperaturdifferenz von ca. 20-25 °C erforderlich. Man muss die Reaktionstemperatur des Reaktors gegenüber den Sumpftemperaturen der verbundenen Kolonnen der EDC-Reinigung deutlich erhöhen, um die Reaktionswärme indirekt an die Naturumlaufverdampfer übertragen zu können. Die Notwendigkeit einer Temperaturerhöhung führt zu einer Verschlechterung der EDC-Ausbeute und zu erhöhter Nebenproduktbildung und vergrößert somit die Menge der abzudestillierenden Substanzen und somit auch die für die Destillation einzusetzende Wärmemenge.

- Die Reaktionswärme des Reaktors kann auch bei relativ niedrigen Reaktionstemperaturen an die Sumpferhitzer der verbundenen EDC-Reinigungskolonnen abgeführt werden, wenn man deren Betriebsdruckniveau absenkt, in der Regel unter Atmosphärendruck. Dies hat bei der Hochsiederkolonne zur Folge, dass Durchmesser und Rücklaufkondensator sehr groß dimensioniert werden müssen, was sich negativ auf die Wirtschaftlichkeit des Verfahrens auswirkt.

- Die Schriften EP 0 075 742 B1 und DE 41 33 810 A1 sehen wie die vorliegende Erfindung eine Nutzung des flüssigen EDC aus dem Direktchlorierungsreaktor vor, eine Nutzung der Enthalpie des dampfförmigen EDC ist nicht möglich.

- Die Schriften DE 196 41 562 und DE 36 04 968 sehen wie die Erfindung die Nutzung des dampfförmigen EDC vor, nicht jedoch eine Nutzung des flüssigen EDC.

**Aufgabe der Erfindung** ist es daher, die als Wärme freiwerdende Reaktionsenthalpie in optimaler Weise im Rahmen des ausgewogenen VCM-Verfahrens zu nutzen.

Mit einem Verfahren der Eingangs bezeichneten Art wird diese Aufgabe gemäß der Erfindung dadurch gelöst, dass die im Reaktionsraum aus der Reaktion von Chlor mit Ethen freigesetzte und im gebildeten 1,2-Dichlorethan enthaltene Reaktionswärme mittels mindestens eines Anteils dampfförmigen 1,2-Dichlorethans (latente Wärme) und mindestens eines weiteren Anteils flüssigen 1,2-Dichlorethans (fühlbare Wärme) aus dem Reaktionsraum herausgeführt und zur Beheizung zweier Fraktionierkolonnen zur Reinigung 1,2-Dichlorethans von höher als 1,2-Dichlorethan siedenden Verunreinigungen verwendet wird.

Eine Ausgestaltung der Erfindung sieht den Einsatz von Fallfilmverdampfern als Sumpfaufheizer der mit der Reaktionswärme der Direktchlorierung beheizten Destillationskolonnen der EDC-Reinigung vor. Fallfilmverdampfer zeichnen sich dadurch aus, dass sie sowohl mit großen als auch mit theoretisch beliebig kleinen Temperaturdifferenzen betrieben werden können. Beim Fallfilmverdampfer strömen zu erhitzende Flüssigkeit und Brüden auf der Rohrseite des Wärmeaustauschers im senkrecht angeordneten Rohrbündel im Gleichstrom nach unten, wobei die Flüssigkeit an der Rohrinnenwand als Rieselfilm nach unten abläuft und die im Rohr gebildeten Brüden im Kembereich des Rohrquerschnittes nach unten abgeleitet werden. Eine entscheidende Rolle für den störungsfreien Betrieb spielt die gleichmäßige und ausreichende Benetzung der Innenrohrheizfläche mit Filmflüssigkeit. Es zeigte sich, dass die Trennwirkung bei den geringen Wärmestromdichten dergestalt verbessert wird, dass bei vorgegebenem Wärmestrom ein größerer Anteil der leichterflüchtigen Komponente in die Dampfphase übergeht.

Dies ist vor allem für den Betrieb der Vakuumkolonne ein ganz wichtiger Aspekt, da auf diese Weise der Anteil an sonst verlorenem EDC im Sumpfabzug der Vakuumkolonne reduziert wird. Eine weitere Ausgestaltung des erfindungsgemäßen Verfahrens sieht daher vor, die Wärme zur Voreindickung und zur Nacheindickung der in den Fraktionierkolonnen anfallenden Sumpfprodukte, nämlich den höher als 1,2-Dichlorethan siedenden Verunreinigungen des 1,2-Dichlorethans, einzusetzen. Hierbei kann der Direktchlorierungsreaktor im Hinblick auf eine schonende Reaktionsführung als Siedereaktor bei einer Verdampfungstemperatur von ca. 110 °C betrieben werden, was zum Betrieb sowohl einer üblichen Hochsiederkolonne als auch einer üblichen Vakuumkolonne beim Einsatz von Fallfilmverdampfem möglich ist.

Eine besondere Ausgestaltung der Erfindung besteht darin, dass ein EDC-Teilstrom, der durch Nutzung der fühlbaren bzw. latenten Wärme abgekühlt wurde, in den Reaktionsraum zurückgeführt wird und dabei zur Unterstützung der Strömung im Reaktionsraum verwendet wird.

In einer weiteren Ausgestaltung der Erfindung wird die Auflösung des in der Direktchlorierung eingesetzten Chlors in einem kleinen, gekühlten EDC-Seitenstrom vorgesehen, wobei die Kühlung bewirkt, dass sich mehr Chlor in EDC lösen kann bzw. dass man weniger EDC zur Auflösung von Chlor benötigt. Hierbei ist es ein Vorteil der Erfindung, dass die in diesem EDC-Seitenstrom enthaltene Wärme zu einem großen Teil zuvor zum Betreiben einer EDC-Reinigungskolonne, vorzugsweise der Vakuumkolonne zum Eindicken von Hochsiedern, genutzt werden kann, so dass die aufzuwendende Kühlmittelmenge zur Abkühlung des Seitenstroms zur Auflösung von Chlor entsprechend geringer wird.

Ein weiterer Vorteil der Erfindung besteht darin, dass die Teilungen in mehrere dampfförmige und flüssige Teilströme mit nicht fixiertem Verhältnis die Anzahl der Freiheitsgrade für die Regelungen der Kolonnen vergrößern und somit die Regelung des Systems mit weniger Einschränkungen versehen ist.

In weiterer Ausgestaltung der Erfindung wird vorgesehen, den nach Nutzung der latenten Wärme als Kondensat anfallenden Strom, der jedoch noch gas- und dampfförmige Komponenten enthalten kann, in einem Nachkondensator weiter zu kondensieren. Bei der Nachkondensation der EDC-Brüden werden Inertgas (Inertgas im Sinne der Reaktionsgleichung (1)) und überschüssiges Ethen nicht kondensiert, zum Teil besteht das Inertgas aus Sauerstoff, der zwecks Inhibierung von Nebenreaktionen zu Reaktion (1) in die Direktchlorierung gegeben oder mit dem eingesetzten Chlor eingetragen und von dort zusammen mit Brüden ausgetragen wurde. Zusammen mit restlichem, dampfförmigen EDC und überschüssigem Ethen kann sich dabei ein explosives Gemisch bilden, da während des Kondensationsvorgangs der Partialdruckanteil der Inertgase gegenüber EDC kontinuierlich steigt Eine weitere Ausgestaltung der Erfindung sieht daher die Messung des Sauerstoffgehalts im Abgas vor. Dieser Messwert wird dazu benutzt, die Nachkondensation des EDC derart zu regeln, dass kein explosives Gemisch entstehen kann, gleichzeitig dem Gas-EDC-Dampfgemisch jedoch ein Maximum an latenter Wärme entzogen werden kann.

Zur Lösung der erfindungsgemäßen Aufgabe sieht die Erfindung eine Vorrichtung zur Nutzung der Reaktionswärme bei der Herstellung von EDC aus Ethen und Chlor vor, die sich auszeichnet durch eine Hochsiederkolonne und eine nachgeschaltete Vakuumkolonne, denen jeweils ein Fallfilmverdampfer zugeordnet ist, wobei der der Hochsiederkolonne zugeordnete Fallfilmverdampfer mit EDC-Dampf aus dem Direktchlorierungsreaktor beheizt und der der Vakuumkolonne zugeordnete Fallfilmverdampfer mit flüssigem EDC aus dem Direktchlorierungsreaktor beheizt wird.

Zur Einbringung gasförmigen Chlors in den Direktchlorierungsreaktor kann wenigstens eine EDC-Teilstromleitung in Verbindung mit einer Leitung zur Beaufschlagung eines Injektors zur Ansaugung gasförmigen Chlors und eine Zuführleitung zur Einbringung des mit Chlor beladenen EDC-Stroms in eine Reaktionsstrecke des Direktchlorierungsreaktors vorgesehen sein.

Alternativ oder in Ausgestaltung hierzu sieht die Erfindung zur Unterstützung eines Naturumlaufes oder zur Ausbildung eines Zwangsumlaufes eine EDC-Teilstromleitung zur Einbringung gekühlter EDC-Ströme aus Leitungen aus den Fallfilmverdampfem zur Beschleunigung des Umlaufes über eine Feinstrahldüse im Fallrohr des Direktchlorierungsreaktors vor.

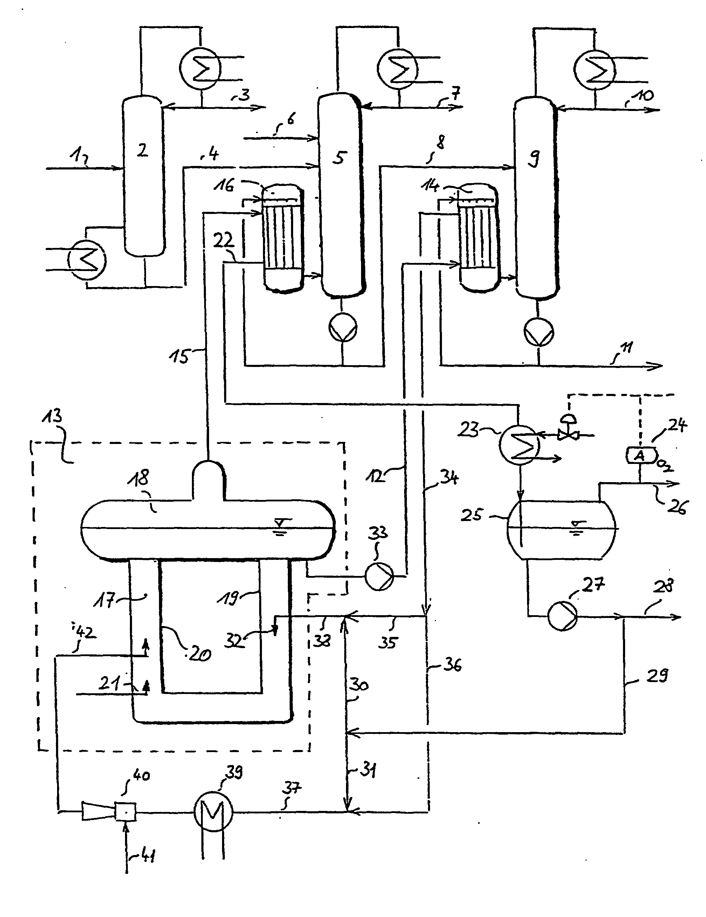

Eine beispielhafte Ausführungsform des Verfahrens der Erfindung sowie der erfindungsgemäßen Vorrichtungen wird in Verbindung mit dem Fließschema gemäß der einzigen Figur zusammen mit Funktionen der einzelnen Elemente näher erläutert, wobei das Verfahren auf diese Ausführungsform nicht beschränkt ist:

In der EDC-Reinigung werden das EDC aus der Oxichlorierung und das nicht umgesetzte EDC aus der EDC-Pyrolyse in einer energieaufwendigen EDC-Destillation gereinigt. Falls erforderlich kann auch das in der Direktchlorierung erzeugte Rein-EDC destillativ gereinigt werden. Das Roh-EDC 1 aus der Oxichlorierung, die in der Figur nicht dargestellt ist, wird in einer Leichtsiederkolonne 2 zunächst von Wasser und Leichtsiedem getrennt, die über die Leichtsiederleitung 3 abgeführt werden. Danach wird das als Sumpfprodukt der Leichtsiederkolonne erhaltene EDC, welches noch Hochsieder enthält, über die Leitung 4 der Hochsiederkolonne 5 zugeführt. Das nicht umgesetzte EDC aus der EDC-Pyrolyse enthält ebenfalls Hochsieder und wird über Leitung 6 der Hochsiederkolonne 5 zugeführt.

In der Hochsiederkolonne 5 werden die zugeführten Stoffströme fraktioniert. Gereinigtes EDC wird am Kopf der Hochsiederkolonne 5 über Leitung 7 abgezogen und als reines EDC gewonnen. Im Sumpf der Hochsiederkolonne 5 reichem sich die Hochsieder an. Die Reinigung des Sumpfproduktes der Hochsiederkolonne 5 wird verstärkt, indem der Sumpfstrom über die Leitung 8 einer Vakuumkolonne 9 zugeführt wird. Gereinigtes EDC wird am Kopf der Vakuumkolonne 9 über Leitung 10 abgezogen und als reines EDC gewonnen. Der Sumpfabzug 11 der Vakuumkolonne 9 besteht aus Hochsiedem und einem kleinen Restanteil EDC.

Die Beheizung der Kolonnen 5 und 9 erfolgt dabei folgendermaßen: Über die Leitung 12 wird ein EDC-Flüssigkeitsstrom aus dem Direktchlorierungsreaktor 13 abgezogen und an den Fallfilmverdampfer 14 der Vakuumkolonne 9 als Heizmedium zu- und abgeführt. Über die Brüdenleitung 15 wird ein EDC-Dampfstrom aus dem Direktchlorierungsreaktor 13 zum Fallfilmverdampfer 16 der Hochsiederkolonne 5 als Heizmedium zugeführt. In den Fallfilmverdampfern 14 und 16 fließt die zu erhitzende Flüssigkeit vom Kopf des Verdampferkörpers als gleichmäßig verteilter, siedender Film auf Grund der Schwerkraft auf der Innenseite der Heizrohre herab und verdampft dabei teilweise.

Mit einem Fallfilmverdampfer kann pro Apparateeinheit eine deutlich größere Wärmeübertragungsfläche realisiert werden als mit den üblicherweise sonst eingesetzten Naturumlaufverdampfern. Dies bedeutet, dass bei großer Anlagenleistung die Sumpfbeheizung beider Kolonnen 5 und 9 mit nur je einer einzigen Fallfilmverdampfereinheit vorgenommen werden kann, während mit einem Naturumlaufverdampfer mehrere Einheiten notwendig wären.

Die Reaktion im als Schlaufenreaktor ausgeführten Direktchlorierungsreaktor 13, der aus einer Reaktionsstrecke 17, einem Ausdampfgefäß 18, einem Fallrohr 19, einem Steigrohr 20, einer Ethen-Eindüsungsstelle 21 und mehreren Eingabestellen für flüssiges EDC besteht, sowie die Auskopplung der Reaktionswärme werden in folgender Weise vorgenommen: Längs der Reaktionsstrecke 17 reagieren gelöstes Chlor und gelöstes Ethen in flüssiger Phase zu EDC, welches teilweise im Ausdampfbehälter 18 verdampft.

Durch die Brüdenleitung 15 wird dampfförmiges EDC dem Fallfilmverdampfer 16 zugeführt, der zur Beheizung der Hochsiederkolonne 5 dient. Hierbei kondensiert der größte Teil des EDC-Dampfes. Der Austrittstrom 22 des Fallfilmverdampfers 16 wird einem Trimmkondensator 23 zugeführt, der zur Regelung des Systems dient. Hierbei muss darauf geachtet werden, dass sich während der Kondensation kein explosives Gemisch zwischen Sauerstoff, restlichem Ethen und EDC-Dampf bilden kann. Daher misst ein Sauerstoffmessgerät 24 den Sauerstoffgehalt und eine damit verbundene Regelungseinrichtung regelt die Zulaufmenge an Kühlmedium des Trimmkondensators 23 entsprechend, es können aber auch noch weitere Regelungseinrichtungen auf den Trimmkondensator 23 aufgeschaltet werden.

Flüssiges EDC wird im Anschluss daran in der Vorlage 25 von nichtkondensierbaren Anteilen getrennt, die durch die Abgasleitung 26 einer weiteren Behandlung zugeführt werden. Aus der Vorlage 25 wird ein EDC-Teilstom 28 mittels der Pumpe 27 als Produktstrom zur VCM-Herstellung, alternativ als Verkaufs-EDC, abgeführt. Der andere EDC-Teilstrom 29, der aufgrund der Kondensation leicht abgekühlt ist, wird aufgeteilt in die EDC-Teilströme 30 und 31. Der eine EDC-Teilstrom 30 wird in das Fallrohr 19 des Direktchlorierungsreaktors 13 zurückgeführt und kann dort durch seinen Impuls als Freistrahl aus einer Düse 32 und auch durch seine gegenüber dem Steigrohr 20 niedrigere Temperatur den Naturumlauf unterstützen. Der andere EDC-Teilstrom 31 wird für die Auflösung von Chlor reserviert.

Aus dem Ausdampfbehälter 18 wird mit der Kreislaufpumpe 33 ein EDC-Strom abgezogen und dem Fallfilmverdampfer 14 zur Beheizung der Vakuumkolonne 9 zugeführt. Der nach Abgabe fühlbarer Wärme abgekühlte EDC-Strom 34 wird aufgeteilt in die EDC-Teilströme 35 und 36. Der eine EDC-Teilstrom 35 wird in das Fallrohr 19 des Direktchlorierungsreaktors 13 zurückgeführt und kann dort durch seinen Impuls als Freistrahl aus der Düse 32 und auch durch seine gegenüber dem Steigrohr 20 niedrigere Temperatur den Naturumlauf unterstützen.

Der andere EDC-Teilstrom 36 wird für die Auflösung von Chlor reserviert. Hierbei ist es möglich, sowohl die EDC-Teilströme 31 und 36 als EDC-Strom 37 als auch die EDC-Teilströme 30 und 35 als EDC-Strom 38 zusammenzufassen, wie in der Figur dargestellt.

Die EDC-Teilströme 31 und 36 werden zusammengefasst als EDC-Strom 37 einem EDC-Kühler 39 zugeführt, wo eine weitere Abkühlung des EDC stattfindet, darauf folgend wird in einem Injektor 40 die leichter lösliche Gaskomponente Chlor 41 angesaugt und aufgelöst. Der mit Chlor beladene EDC-Strom 42 wird dann der Reaktionsstrecke 17 zugeführt.

Zur Veranschaulichung dient das folgende Zahlenbeispiel auf der Basis einer Simulationsrechnung: Im als Schlaufenreaktor ausgeführten Direktchlorierungsreaktor 13 werden 4 765 kg/h Ethylen (170 kmol/h) mit der äquimolaren Menge Chlor umgesetzt. Die Temperatur im Ausdampfgefäß 18 beträgt 110 °C, der Druck 2,1 bar (absolut). Über die Brüdenleitung 15 werden 70 465 kg/h dampfförmiges EDC dem Fallfilmverdampfer 16 zugeführt, der eine Leistung von 5 094 kW an die Hochsiederkolonne 5 abgibt. Die Sumpftemperatur der Hochsiederkolonne 5 beträgt 100 °C. Die Restmenge EDC wird im Trimmkondensator 23 mit Kühlwasser kondensiert, wobei eine Wärmeleistung von 951 kW abgeführt wird.

Der Austrittstrom der Kondensationsstrecke mit einer Temperatur von 102 °C wird in der Vorlage 25 gesammelt. Hier wird auch der mit EDC beladene Abgasstrom 26 von 2 848 kg/h abgetrennt. Die Pumpe 27 fördert einerseits den EDC-Teilstrom 28 von 14 017 kg/h zur Weiterverarbeitung. Andererseits fördert die Pumpe 27 den EDC-Teilstrom 29, der in diesem Fall gleich dem EDC-Teilstrom 30 ist, von 53 600 kg/h zurück in das Fallrohr 19 des Schlaufenreaktors.

Aus dem Ausdampfbehälter 18 wird durch die Pumpe 33 der EDC-Strom 12 von 250 000 kg/h abgezogen und dem Fallfilmverdampfer 14 der Vakuumkolonne 9 zugeführt. Der Fallfilmverdampfer 14 gibt eine Wärmeleistung von 1 814 kW an die Vakuumkolonne 9 ab, deren Sumpftemperatur 87 °C beträgt. Der abgekühlte EDC-Strom 34 am Austritt des Fallfilmverdampfers 14 hat eine Temperatur von 92°C.

Ein Anteil von 40 % dieses Stroms wird als EDC-Teilstrom 35 abgezweigt und ebenfalls zurück in das Fallrohr 19 des Direktchlorierungsreaktors 13 gegeben. Die verbleibenden 60 % (150 000 kg/h) werden als EDC-Strom 37, der in diesem Fall gleich dem EDC-Teilstrom 36 ist, im Kühler 39 auf 45 °C abgekühlt und saugen im Injektor 40 eine Menge von 12 100 kg/h Chlor an. Dabei erwärmt sich der EDC-Strom 41 durch die beträchtliche Lösungswärme des Chlors wieder auf 75 °C. Dieser EDC-Strom 41 wird in die Reaktionsstrecke 17 eingebracht, wobei zweckmäßigerweise ein strömungsunterstützendes Düsensystem verwendet wird.

**Liste der verwendeten Bezugszeichen**

| | |
|---|---|
| 1 | Roh-EDC |
| 2 | Leichtsiederkolonne |
| 3 | Leichtsiederleitung |
| 4 | Leitung |
| 5 | Hochsiederkolonne |
| 6 | Leitung |
| 7 | Leitung |
| 8 | Leitung |
| 9 | Vakuumkolonne |
| 10 | Leitung |
| 11 | Sumpfabzug |
| 12 | Leitung |
| 13 | Direktchlorierungsreaktor |
| 14 | Fallfilmverdampfer |
| 15 | Brüdenleitung |
| 16 | Fallfilmverdampfer |
| 17 | Reaktionsstrecke |
| 18 | Ausdampfgefäß |
| 19 | Fallrohr |
| 20 | Steigrohr |
| 21 | Ethen-Eindüsungsstelle |
| 22 | Austrittstrom |
| 23 | Trimmkondensator |
| 24 | Sauerstoffmessgerät |
| 25 | Vorlage |
| 26 | Abgasleitung |
| 27 | Pumpe |
| 28 | EDC-Teilstrom |
| 29 | EDC-Teilstrom |
| 30 | EDC-Teilstrom |
| 31 | EDC-Teilstrom |
| 32 | Düse |
| 33 | Kreislaufpumpe |
| 34 | EDC-Strom |
| 35 | EDC-Teilstrom |
| 36 | EDC-Teilstrom |
| 37 | EDC-Strom |
| 38 | EDC-Strom |
| 39 | EDC-Kühler |
| 40 | Injektor |
| 41 | Chlor |
| 42 | EDC-Strom |

**Patentansprüche**

**1.** Verfahren zur Nutzung der Reaktionswärme bei der Herstellung von 1,2-Dichlorethan aus Ethen und Chlor, **dadurch gekennzeichnet, dass** die im Reaktionsraum aus der Reaktion von Chlor mit Ethen freigesetzte und im gebildeten 1,2-Dichlorethan enthaltene Reaktionswärme mittels mindestens eines Anteils dampfförmigen 1,2-Dichlorethans (latente Wärme) und mindestens eines weiteren Anteils flüssigen 1,2-Dichlorethans (fühlbare Wärme) aus dem Reaktionsraum herausgeführt und zur Beheizung zweier Fraktionierkolonnen zur Reinigung 1,2-Dichlorethans von höher als 1,2-Dichlorethan siedenden Verunreinigungen verwendet wird.

**2.** Verfahren nach Anspruch 1, **dadurch gekennzeichnet, dass** die Beheizung der beiden Fraktionierkolonnen zur Reinigung 1,2-Dichlorethans von höher als 1,2-Dichlorethan siedenden Verunreinigungen mittels Fallfilmverdampfern vorgenommen wird.

**3.** Verfahren nach einem der vorangegangenen Ansprüche, **dadurch gekennzeichnet, dass** die Wärme zur Voreindickung und zur Nacheindickung der in den Fraktionierkolonnen anfallenden Sumpfprodukte, welche die höher als 1,2-Dichlorethan siedenden Verunreinigungen des 1,2-Dichlorethans darstellen, eingesetzt wird.

**4.** Verfahren nach einem der vorangegangen Ansprüche, **dadurch gekennzeichnet, dass** mindestens ein nach Nutzung latenter Wärme kondensierter Teilstrom des 1,2-Dichlorethans in das Reaktionsgefäß zurückgeführt und dort zur Unterstützung der Strömung im Reaktionsgefäß verwendet wird.

**5.** Verfahren nach einem der Ansprüche 1 bis 3, **dadurch gekennzeichnet, dass** mindestens ein nach Nutzung fühlbarer Wärme abgekühlter Teilstrom des 1,2-Dichlorethans in das Reaktionsgefäß zurückgeführt und dort zur Unterstützung der Strömung im Reaktionsgefäß verwendet wird.

**6.** Verfahren nach einem der vorangegangen Ansprüche, **dadurch gekennzeichnet, dass** ein nach Nutzung der latenten oder fühlbaren Wärme abgekühlter Teilstrom des 1,2-Dichlorethans weiter abgekühlt wird und danach dazu verwendet wird, Chlor darin aufzulösen und diese Lösung anschließend in das Reaktionsgefäß geführt wird.

**7.** Anlage zur Nutzung der Reaktionswärme bei der Herstellung von 1,2-Dichlorethan aus Ethen und Chlor, **dadurch gekennzeichnet, dass** eine Hochsiederkolonne (5) und eine nachgeschaltete Vakuumkolonne (9), denen jeweils ein Fallfilmverdampfer (16 bzw. 14) zugeordnet ist, wobei der der Hochsiederkolonne (5) zugeordnete Fallfilmvordampfer (16) mit 1,2-Dichlorethan-Dampf aus dem Direktchlorierungsreaktor (13) beheizt und der der Vakuumkolonne (9) zugeordnete Fallfilmverdampfer (14) mit flüssigem 1,2-Dichlorethan aus dem Direktchlorierungsreaktor (13) beheizt wird.

**8.** Anlage nach Anspruch 7, **gekennzeichnet durch** wenigstens eine 1,2-Dichlorethan-Teilstromleitung (36 bzw. 31) in Verbindung mit einer Leitung (29) zur Beaufschlagung eines Injektors (49) zur Ansaugung gasförmigen Chlors (Leitung 41) und eine Zuführleitung (42) zur Einbringung des mit Chlor beladenen EDC-Stromes in einen Reaktionsstrecke (17) des Direktchlorierungsreaktors (13).

**9.** Anlage nach Anspruch 7 oder 8, **gekennzeichnet durch** eine EDC-Teilstromleitung (38) zur Einbringung gekühlter EDC-Ströme aus Leitungen (35, 30) aus den Fallfilmverdampfern (14, 16) zur Beschleunigung des Umlaufes über eine Düse (32) im Fallrohr (19) des Direktchlorierungsreaktors (13)

**Claims**

**1.** Process for the recovery of the reaction heat developed in the production of 1,2-dichloroethane from ethene and chlorine,
**characterised in that** the heat released in the reactor chamber by the reaction of chlorine with ethene and of the reaction heat contained in the 1,2-dichloroethane is recovered from the reaction chamber by means of at least one portion of vaporous 1,2-dichloroethane (latent heat) and at least one more portion of liquid 1,2-dichloroethane (sensible heat) so that said heat can be exploited for heating two fractionation columns required to remove from the 1,2-dichloroethane any impurity which is higher boiling than the 1,2-dichloroethane itself.

**2.** Process according to claim 1,
**characterised in that** falling film evaporators are deployed to heat the two fractionation columns used for removing impurities from 1,2-dicholoethane, said impurities being higher boiling that 1,2-dichloroethane.

**3.** Process according to any of the preceding claims,
**characterised in that** heat is used to pre-concentrate and post-concentrate the bottom products obtained in the fractionation columns, i.e. the impurities in the 1,2-dichloroethane which are higher boiling than the 1,2-dichloroethane itself.

**4.** Process according to any of the preceding claims,
**characterised in that** at least one part stream of 1,2-dichloroethane condensed by the recovery of latent heat is returned to the reaction chamber to enhance the flow inside the reaction chamber.

5. Process according to one of claims 1 to 3,
   **characterised in that** at least one part stream of 1,2-dichloroethane cooled by the recovery of sensible heat is returned to the reaction chamber to enhance the flow inside the reaction chamber.

6. Process according to any of the preceding claims,
   **characterised in that** a part stream of 1,2-dicholoroethane cooled by the recovery of the latent and sensible heat is further cooled and then used to dissolve chlorine, this solution being passed to the reaction chamber.

7. Device for the recovery of the reaction heat obtained in the production of 1,2-dichloroethane from ethene and chlorine,
   **characterised in that** heavy ends column 5 and downstream vacuum column 9 are equipped with one falling film evaporator each (16 or 14), falling film evaporator 16 assigned to heavy ends column 5 being heated with 1,2-dichloroethane vapour from direct chlorination reactor 13 and falling film evaporator 14 assigned to vacuum column 9 being heated with liquid 1,2-dichloroethane from direct chlorination reactor 13.

8. Device according to claim 7,
   **characterised in that** provision is made for at least one 1,2-dichloroethane part stream line (36 or 31) in conjunction with line 29 to injector 49 for the intake of gaseous chlorine (line 41) and a line 42 for feeding the chlorine-bearing EDC stream to reaction section 17 of direct chlorination reactor 13.

9. Device according to claim 7 or 8,
   **characterised in that** EDC part stream line 38 is installed for feeding cooled EDC streams from lines 35 and 30 of falling film evaporators 14 and 16 so that the circulation velocity increases with the aid of nozzle 32 arranged in downpipe 19 of direct chlorination reactor 13.

## Revendications

1. Procédé pour la récupération de la chaleur obtenue au cours de la production de 1,2-dichloréthane à partir du chlore et de l'éthène,
   **caractérisé en ce que** la chaleur de réaction dégagée dans la chambre de réaction par suite de la réaction du chlore avec l'éthène et contenue dans le 1,2-dichloréthane formé est exploitée de la manière suivante: au moins une portion de 1,2-dichloréthane vaporeux (chaleur latente) et au moins une portion de 1,2-dichloréthane liquide (chaleur sensible) sont soutirées de la chambre de réaction pour être utilisées comme agent de chauffage pour les deux colonnes de fractionnement afin de séparer le 1,2-dichloréthane des impuretés avec un point d'ébullition plus élevé que celui du 1,2-dichloréthane.

2. Procédé selon la revendication 1,
   **caractérisé en ce que** le chauffage des deux colonnes à fractionnement à fin de séparer le 1,2-dichloréthane des impuretés avec un point d'ébullition plus élevé que celui du 1,2-dichloréthane se fait à l'aide des évaporateurs à film tombant.

3. Procédé selon une des revendications précédentes,
   **caractérisé en ce que** la chaleur est utilisée pour la concentration primaire et secondaire des produits du bas des colonnes de fractionnement, c.-à-d. les impuretés ayant un point d'ébullition plus élévé que celui du 1,2-dichloréthane.

4. Procédé selon une des revendications précédentes,
   **caractérisé en ce qu'**au moins un flux partiel de 1,2-dichloréthane condensé au bout du cycle de l'utilisation de la chaleur latente est recyclé vers l'appareil de réaction pour y intensifier les flux dans le dit appareil.

5. Procédé selon la revendication No. 1 ou 3,
   **caractérisé en ce qu'**au moins un flux partiel de 1,2-dichloréthane condensé au bout du cycle de l'utilisation de la chaleur sensible est recyclé vers l'appareil de réaction pour y intensifier les flux dans le dit appareil.

6. Procédé selon une des revendiactions précédentes,

**caractérisé en ce qu**'un flux partiel de 1,2-dichoréthane refroidi par suite de l'utilisation de la chaleur latente ou sensible est soumis à un refroidissement supplémentaire pour être alors mis en oeuvre pour la dissolution du chlore et pour retourner cette solution vers l'appareil de réaction.

7. Dispositif pour la récupération de la chaleur lors de la production de 1,2-dichloréthane à partir de l'éthène et du chlore,
**caractérisé en ce que** le dit dispositif est doté d'une colonne à fraction de haut point d'ébullition (5) et d'une colonne à vide (9) disposée en aval, chacune munie d'un évaporateur à film tombant (16 ou 14), l'évaporateur (16) de la colonne à fraction de haut point d'ébullition (5) étant chauffé par vapeur de 1,2-dichloréthane originaire du réacteur à chlorination directe (13) et l'évaporateur (14) de la colonne à vide (9) par 1,2-dichloréthane liquide originaire du réacteur de chlorination directe (13).

8. Dispositif selon la revendication No. 7,
**caractérisé en ce qu**'au moins une conduite de flux partiel de 1,2-dichloréthane (voir No. 36 ou 31), conjointement avec une conduite d'amenée (29) vers un injecteur (49) pour l'aspiration du chlore gazeux (conduite 41) et une conduite d'amenée (42) pour le flux EDC chargé de chlore et introduit dans la gaine de réaction (17) du réacteur à chlorination directe (13).

9. Dispositif selon la revendication No. 7 ou 8,
**caractérisé en ce qu**'une conduite de flux partiel EDC (38) est monté pour l'amenée des flux EDC refroidis par les conduites (35, 30) venant des évaporateurs à film tombant (14, 16) en vue d'accélérer la circulation par une buse (32) placée dans le tuyau de descente (19) du réacteur à chlorination directe (13).